# EUROPEAN PATENT APPLICATION

(11) **EP 2 944 372 A1**
(43) Date of publication of application: **18.11.2015**
(21) Application number: 14168756.6
(22) Date of filing: 17.05.2014
(51) Int. Cl.: B01F 11/00, B01F 13/08, B01F 15/00, B01F 15/02, G01N 1/38, C12M 1/06

(54) **Method and device for suspending cells**

(71) Applicant: Miltenyi Biotec GmbH, 51429 Bergisch Gladbach (DE)
(72) Inventor: Peters, Ralf-Peter, 51467 Bergisch Gladbach (DE); Büscher, Martin, 51429 Bergisch Gladbach (DE); Ignatius, Sven, 51465 Bergisch Gladbach (DE)
(74) Representative: Kisters, Michael Marcus

(57) **Abstract**

The invention is related to a method for suspending particles in a fluid characterized in generating at least two streams of fluid moving in circles with opposite direction wherein the streams have at least one common zone of turbulence by moving a rod non-flexible mixing rod (4) through the fluid.

Such suspensions can be provided by a mixing device comprising a first magnet (1) rotating around a first axis (2); a second magnet (3) attached to a non-flexible mixing rod (4); a joint (6) that allows movement of the second magnet (3) about a second axis (5); whereby rotation of the first magnet (1) about the first axis (2) drives movement of the second magnet (3) and mixing rod (4) about the second axis (5).

## Description

This invention relates to a method and a device for suspending particles in a fluid, especially for suspending living cells in small volume of fluids.

### Background

Biological samples like cells need to be suspended in a liquid for culturing or analyzing purposes. Such suspensions are stable for short time until the sample starts sedimentation which makes remixing necessary. Mixing suspensions of biological samples is a long known technique for which a various devices are commercially available. Design and function of the mixing devices depend on the nature and volume of the sample to be mixed, the size and form of the mixing vessel and subsequent processing steps of the sample.

Besides mixing a suspension by a stirrer coupled to an electric motor, magnetic cell stirrers are in common use. To provide larger volumes of suspensions, US 3854704 and US 3572651 disclose a magnetic cell stirrer wherein the suspension is mixed by a rotating magnet placed in the mixing vessel via a flexible shaft. The magnet inside of the vessel is forced into rotational movement by a magnet outside of the vessel which in turn is rotated by a conventional magnetic stirring apparatus. The flexible shaft is attached to the mixing vessel and can not be removed or inserted in the vessel.

US 4204774 describes a magnetic cell stirrer wherein the suspension is mixed by a rotating stirring rod. The rod is rotated in a circular path in the mixing vessel by an eccentric means and a synchronous motor. The magnetic cell stirrer is firmly attached to the mixing vessel and can not be removed or inserted in the vessel.

Biological samples for testing or analyzing purposes are usually provided in small volumes ranging from less than 10 µl to 5 ml. For example, commonly used microplates provide up to 1024 wells having a volume less than one milliliter. Mixing of such small volumes by mere stirring is difficult due to capillary forces and a low surface to volume ratio of the vessel resulting in adhesion of the liquid to the vessel walls. Mixing by stirring generates a single stream of liquid moving in a circle adjacent to the vessel wall having low turbulence and accordingly a low mixing efficiency.

Suspensions in microplate are therefore mixed by shaking or vibrating the whole microplate i.e. all wells simultaneously. Shaking cell suspensions in a microplate might result in cell loss since cells can adhere to walls of a well above the surface of the liquid

It would be beneficial to provide small volume suspensions of biological samples without stirring the fluid in circles and/or the need to shake/vibrate other vessels. It would furthermore be desirous to provide a device to generate such suspensions which can be implemented in analytical devices like a FACS machines for sample preparation upstream of the cell analysis device.

Surprisingly, it was found that particle suspensions especially in small volume containers can be efficiently provided by generating at least two convection currents of fluid moving in circles with opposite directions rather than creating a single circular stream by stirring.

### Object of the invention

Accordingly, object of the invention is a method for suspending particles in a fluid characterized in generating at least two convection currents of fluid moving in circles with opposite direction wherein the currents have at least one common zone of turbulence by moving a rod non-flexible mixing rod (4) through the fluid.

With the method of the invention, it is possible to suspend particles even in small volumes of fluid without loss of particles or fluid due to spilling or re-agglomeration .

Another object of the invention is a mixing device comprising: a first magnet (1) rotating around a first axis (2); a second magnet (3) attached to a non-flexible mixing rod (4); a joint (6) that allows movement of the second magnet (3) about a second axis (5); whereby rotation of the first magnet (1) about the first axis (2) drives movement of the second magnet (3) and mixing rod (4) about the second axis (5).

Within the scope of the invention, the terms "mixing" or "suspending" are synonym and are directed to provide a suspension of particles in a fluid.

The method and device according to the invention is especially useful for suspending particles like a biological sample in a liquid or fluid. "Biological sample" can be any type of tissue or cells to be suspended in an appropriate fluid like cell nutrition buffer. The cells should be suspended in the liquid without physically damage.

### Brief description of the drawings:

- Fig. 1: shows the device with first magnet (1), first axis (2), second magnet (3), non-flexible mixing rod (4), joints (6, 6') and second axis (5).
- Fig. 2: shows the first magnet (1) mounted on electric motor (8) with counter weight (9) rotating around axis first (2')
- Fig. 3: shows second magnet (3) attached to the holder of non-flexible mixing rod (4); hinged to joints (6, 6'). The joints are fixed by through holes (7), thereby movement of the holder is limited as depicted by dashed line 6". Mixing rod (4) is guided by shaft (10).
- Fig. 4: side view of the mixing system with electric motor (8) and mixing rod (4)
- Fig. 5: front view of the mixing system with the hinged joints (6, 6') for the non-flexible mixing rod (4) removed. Mixing rod (4) is guided by holder or shaft (10)
- Fig. 6: figure-8 shaped movement of the non-flexible mixing rod (4) with common zone of turbulence (13)
- Fig. 7: convection currents of fluids created by figure-8 shaped movement of the non-flexible mixing rod (4) with common zone of turbulence (13)
- Fig. 8: Examples of movement of the non-flexible mixing rod (4) (thin line) and resulting convection currents of fluids (bold line)

### Detailed description of the invention

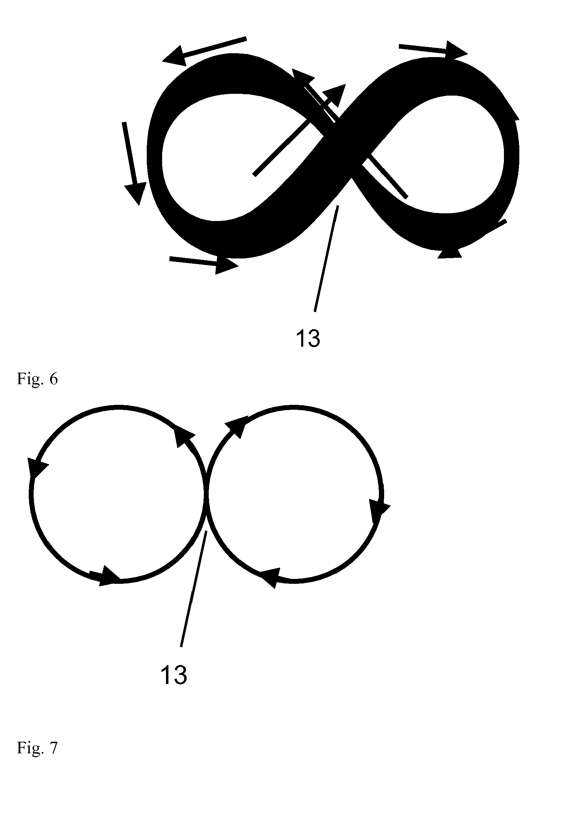

The preferred movements of the non-flexible mixing rod (4) through the fluid are substantially figure-8 shaped or substantially lateral. Substantially figure-8 shaped movement is depicted in Fig. 7 with arrows indicating the direction of movement. By moving the non-flexible mixing rod (4) through the fluid, at least two convection currents of fluid result as shown in Fig. 7 with common zone of turbulence (13). The currents flow with opposite direction and share at least one common zone of turbulence.

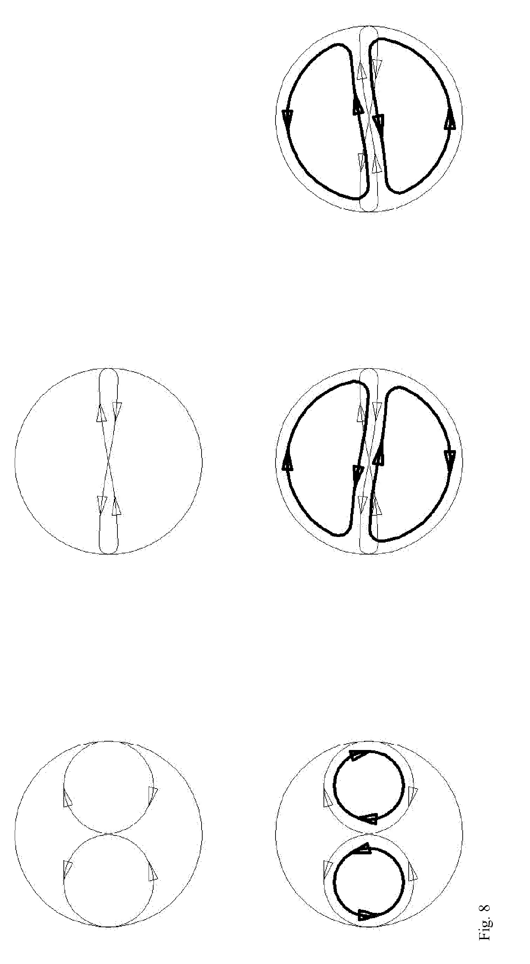

Fig. 8 shows examples of moving paths of the non-flexible mixing rod (4) (thin lines) through the fluid and the resulting convection currents of fluid (bold lines).

The direction of the movement of the non-flexible mixing rod (4) can be the same or can be periodically inverted during the duration of the method. For example, if the movement of the non-flexible mixing rod is substantially figure-8 shaped, the rod may perform a plurality of completed figure-8 shaped turns, be stopped and start figure-8 shaped turns in the opposite direction. Inverting the direction of movement of the rod generates additional turbulence and for better mixing efficiency.

Essential for the mixing device according to the invention is the movement of the mixing rod (4) about the second axis (5). The mixing device can be designed for substantially lateral, substantially figure-8 shaped or substantially circular movement of the rod about the second axis (5).

The term "substantially" means that the movement may deviate slightly from a lateral or circular movement. Preferable, the movement of the mixing rod (4) is substantially lateral or substantially figure-8 shaped about the second axis (5). In quiescent state of the first magnet, the first axis and the second axis are substantially aligned (i.e. in line) to each other. For example, in Fig.8, right and middle drawings show in thin line substantial lateral paths of movements.

The movement of the mixing rod originates from the rotation of the first magnet (1) around first axis (2). This rotational movement is transferred by magnetic interaction between the first magnet (1) and the second magnet (3) to the non-flexible mixing rod (4). Magnetic interaction between the first magnet (1) and the second magnet (3) requires that the distance between the both magnets is sufficiently small. Depending on the orientation of the poles of the first and second magnet, the interaction may generate attracting or opposing forces. In the present invention, first and second magnets are preferable orientated with the same pole facing each other (N-N or S-S). The rotational movement of first magnet (1) and the opposing forces between the magnets "pushes" second magnet (3) into motion.

First magnet (1) and the second magnet (3) are permanent magnets without any special requirements.

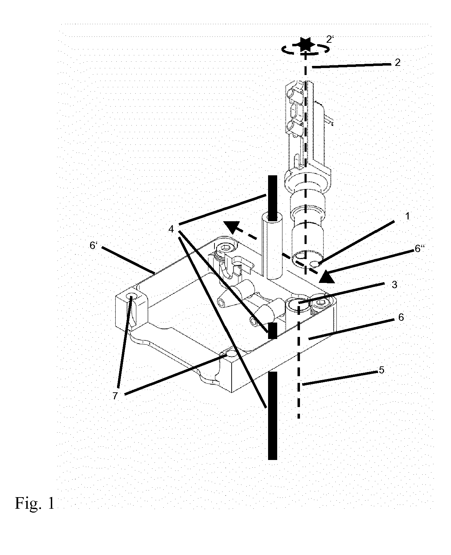

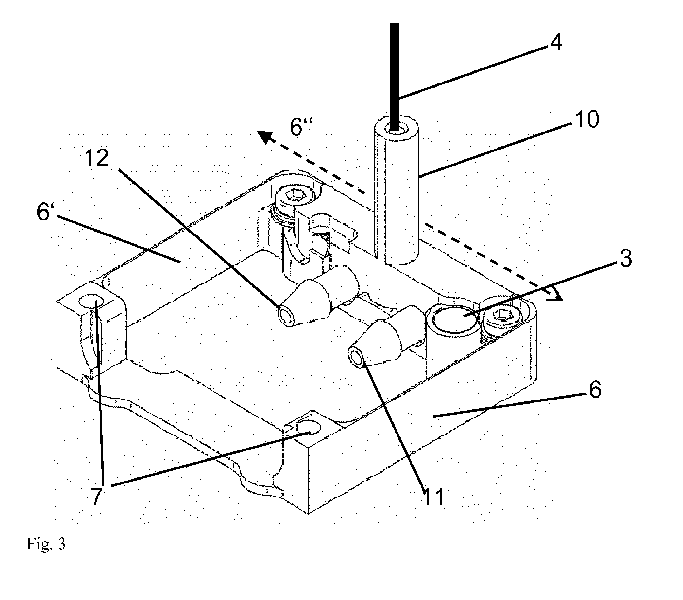

In Fig. 1 and 3, the mixing rod (4) is guided by shaft or guidance (10) which is attached to joint or hinge (6, 6'). The moving path of the non-flexible mixing rod (4) is defined or restricted by the joint (6, 6') that allows movement of the second magnet (3) about a second axis (5). In a preferred embodiment of the invention, joint (6) is provided as holder with one or two sheet-like hinges which can only swing or vibrate joint (6) about the second axis (5). This embodiment is shown in Fig. 3 and results in a substantial lateral movement of the mixing rod (4) along dashed line 6".

A substantially circular of substantially figure-8 shaped movement of the mixing rod may be provided by appropriate formed joint or hinge (6, 6') and/or slotted guide rod and/or sliding blocks.

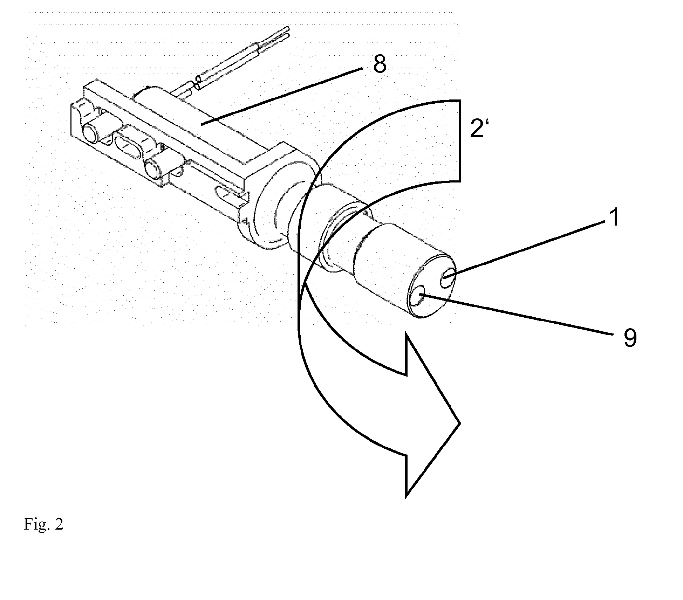

As shown in Fig. 2, rotation of the first magnet (1) is accomplished by an electric motor (8) which rotates around a first axis (2). The first magnet (1) may be fixed in line with the first axis (2) or in a preferred embodiment, asymmetric to the first axis (2) i.e. positioned with a distance of 1 - 5 mm to the first axis (2). In this embodiment, preferable a counterweight (9) to the first magnet (1) is positioned at the same distance to the first axis (2) to prevent imbalance.

The mixing rod (4) is manufactured from a non-flexible material like stainless steel or the like in contrast to flexible materials like polymers, rubber etc. The mixing rod (4) may be solid or provided as tube or cannula.

If provided as tube or cannula, the mixing rod (4) may be used for filling the mixing vessel with the particles and fluids to be mixed/suspended. In another variant of the invention, the particles (biological sample/cells) and/or the fluid are at least in part inserted or removed from the mixing vessel through a mixing rod (4) provided as tube. Particles and fluids may already be premixed before being provided into the mixing vessel. For this purpose, mixing rod (4) is optionally provided with appropriate connectors like the Luer-system for connection with tubing set.

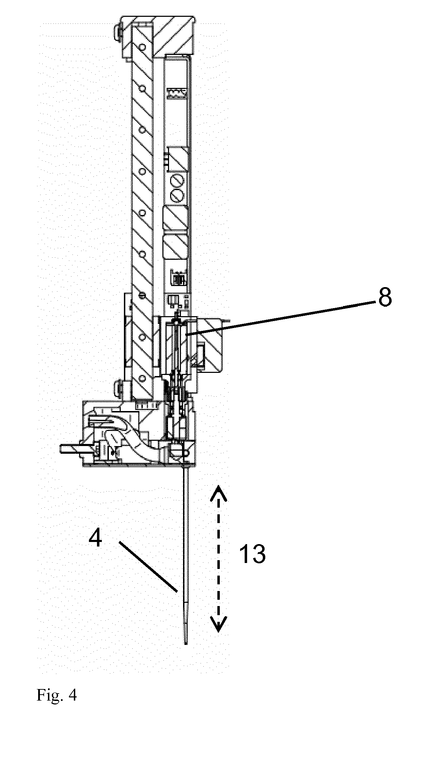

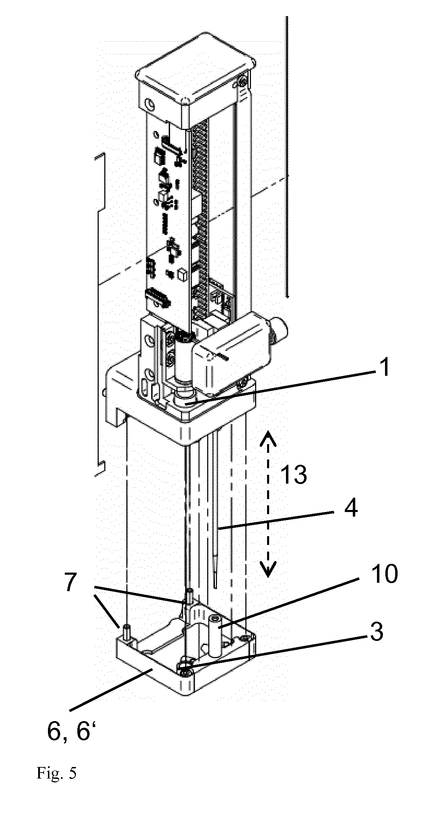

In yet another embodiment of the invention, the mixing device is provided with least one mixing vessel and the mixing rod can be inserted into and withdrawn from the mixing vessel. Inserting/withdrawing of the mixing rod from the vessel requires either moving the mixing vessel relative to a stationary mixing rod or the mixing rod relative to a stationary mixing vessel. This variant is shown in Fig. 4 and 5, with the position of mixing rod (4) being adjustable relative a not shown mixing vessel in direction of dashed line (13).

In a first variant of this embodiment of the invention, the mixing device is provided with at least one mixing vessel and the mixing rod can be inserted in the mixing vessel by moving the mixing vessel relative to a stationary mixing rod. In other words, the mixing vessel is moved up or down for inserting or withdrawing the mixing rod from the vessel.

In a second variant of this embodiment of the invention, the mixing device is provided with at least one mixing vessel and the mixing rod can be inserted in the mixing vessel by moving the mixing rod relative to a stationary mixing vessel. In other words, the mixing rod itself is moved up or down for inserting or withdrawing the mixing rod from the vessel.

As mixing vessel, wells of commercially available microplates are preferred. Of course, the movement of the second magnet (3) and mixing rod (4) about the second axis (5) needs to be adjusted to the space available within the vessel. The mixing rod shall mix the liquid and the biological vessel without touching the walls of the mixing vessel.

The method of the invention is preferable performed in a mixing vessel wherein the ratio of the thickness of the non-flexible mixing rod (4) and the maximum inner width of the mixing vessel is between 0.1 and 0.3.

For example, the mixing rod may have an outer diameter of 1.5 to 0.3 mm and may be utilized in mixing vessels with an maximum inner width of 1 to 10 mm.

The method and device of the invention can be used for mixing or suspending subsequently a plurality of different samples. It is another object of the invention to avoid contamination of a sample to be suspended by traces or drops of a different sample adhering to the mixing rod.

In further embodiments of the invention, the surface of the mixing rod is cleaned or stripped from adhering mixture by a cleaning pod or a cleaning liquid. The cleaning pod may be made from any material suitable for soaking up liquids like cotton or tissue paper. Better cleaning of the mixing rod can be achieved by using a cleaning liquid and washing the mixing rod. In a first variant of this embodiment, the mixing rod is guided through a reservoir of the cleaning liquid. In a preferred variant, the mixing rod is guided through a stream of cleaning liquid, thereby assuring that the mixing rod is only in contact with fresh, uncontaminated cleaning liquid.

Fig. 3 shows this embodiment of the invention, where joint or hinge (6, 6') is provided with shaft or guidance (10) for the mixing rod (4) which can be flushed with cleaning liquid provided and removed through orifices (11) and (12).

The method and device according to the invention is especially useful for sample preparation in automated processing or analyzing of biological samples. Usually, such processing requires a homogenous suspension of the sample in a fluid. At best, the homogenous sample is suspended in the fluid shortly before processing.

Accordingly, another object of the invention is a process for providing a suspension, comprising: placing particles like a biological sample and a fluid in a mixing vessel; placing the mixing rod of the mixing device as described in the mixing vessel and suspending the particles in the liquid/fluid.

The process of the invention can be utilized within high-throughput automates or robots for analyzing a plurality of biological samples. Therefore, multi-well microplates are preferable used as mixing vessel in the process of the invention and the mixing rod is inserted in each well /mixing vessel for mixing and withdrawn after suspending/mixing is completed.

The device and method of the invention can be used for parallel processing of a plurality of samples. For example, suspending of a plurality of biological samples on multi-well microplates may be performed in parallel by 2 to 6 devices of the invention.

The mixing device of the invention can be used for any automated processing or automated analyzing of biological samples in suspension or solution, especially ELIZA or FACS systems.

## Claims

1. Method for suspending particles in a fluid **characterized in** generating at least two convection currents of fluid moving in circles with opposite direction wherein the currents have at least one common zone of turbulence by moving a non-flexible mixing rod (4) through the fluid.

2. Method according to claim 1, **characterized in that** the movement of the non-flexible mixing rod (4) through the fluid is substantially lateral.

3. Method according to claim 1, **characterized in that** the movement of the non-flexible mixing rod (4) through the fluid is substantially figure-8 shaped.

4. Method according to any of the claims 1 to 3, **characterized in that** the direction of movement of the non-flexible mixing rod (4) is periodically inverted.

5. Method according to any of the claims 1 to 4, **characterized in that** the method is performed in a mixing vessel comprising the suspension and that the ratio of the thickness of the non-flexible mixing rod (4) and the maximum inner width of the mixing vessel is between 0.1 and 0.3.

6. Method according to any of the claims 1 to 5 **characterized in that** the mixing rod is inserted and removed from the mixing vessel by moving the mixing rod relative to a stationary mixing vessel.

7. Method according to any of the claims 1 to 5 **characterized in that** the mixing rod is inserted and removed from the mixing vessel by moving the mixing vessel relative to a stationary mixing rod.

8. Method according to any of the claims 1 to 7 **characterized in that** the particles and/or the fluid and/or the suspension are at least in part inserted or removed from the mixing vessel through a mixing rod (4) provided as tube.

9. A mixing device comprising:
a first magnet (1) rotating around a first axis (2); a second magnet (3) attached to a non-flexible mixing rod (4); a joint (6) that allows movement of the second magnet (3) about a second axis (5); whereby rotation of the first magnet (1) about the first axis (2) drives movement of the second magnet (3) and mixing rod (4) about the second axis (5).

10. Mixing device according to claim 9, wherein the movement of the mixing rod is substantially lateral or figure-8 shaped about the second axis (5).

11. Mixing device according to claim 9 or 10, **characterized in that** at least one mixing vessel is provided and the mixing rod can be inserted in the mixing vessel by moving the mixing vessel relative to a stationary mixing rod.

12. Mixing device according to claim 9 or 10, **characterized in that** at least one mixing vessel is provided and the mixing rod can be inserted in the mixing vessel by moving the mixing rod relative to a stationary mixing vessel.

13. Mixing device according to any of the claims 9 to 12, **characterized in that** in quiescent state of the first magnet, the first axis and the second axis are substantially aligned to each other.

14. Mixing device according to any of the claims 9 to 13, **characterized in that** the mixing rod is stripped from adhering mixture by a cleaning pod or a cleaning liquid.

15. Mixing device according to any of the claims 1 to 14 **characterized in that** the mixing rod is a tube.
